# EUROPEAN PATENT APPLICATION

(11) **EP 3 760 185 A1**
(43) Date of publication of application: **06.01.2021**
(21) Application number: 20182472.9
(22) Date of filing: 26.06.2020
(51) Int. Cl.: A61K 8/73, A61Q 19/08

(54) **COSMETIC COMPOSITION TO PREVENT AND DELAY SKIN AGING**

(30) Priority: 01.07.2019 IT 201900010545
(71) Applicant: Riccio, Rodolfo, 80132 Napoli (IT)
(72) Inventor: Riccio, Rodolfo, 80132 Napoli (IT)
(74) Representative: Coppo, Alessandro

(57) **Abstract**

In one aspect, the present invention concerns the use of certain maltodextrins as a cosmetically active ingredient effective in preventing or delaying skin aging. The invention further relates to the cosmetic use of a composition containing maltodextrins to treat skin aging and prevent or reduce facial corrugations.

## Description

### FIELD OF THE INVENTION

The present invention concerns a cosmetic composition to prevent and delay skin aging.

The present invention originates in the cosmetic field.

In particular, the invention relates to the use of specific complex carbohydrates for the cosmetic treatment of the skin to reduce the typical signs due to skin aging and exposure to external agents such as solar radiation.

### STATE OF THE ART

The skin is a multifunctional structure, substantially dedicated to protection from external aggressive chemical, physical and biological agents. Like all tissues of the human body, it is also subject to the natural biological aging process of the human body whereby a reduction in cell turnover occurs. The physiological aging process can be accelerated by exposure to harmful external factors/agents such as sun ultraviolet radiation, environmental pollutants, and smoke. Exposure to these agents results in cellular damage, mainly caused by an excessive production of free radicals and a reduction in the level of antioxidant substances, which causes damage to structural proteins, such as collagen and elastin fibers, and consequent structural changes of the dermis and subcutaneous tissue.

During this process, the water content of the skin is reduced, the production of collagen slows down, and elastin, a protein that allows the skin to restore the physiological conformation after stretching, becomes less reactive.

Skin aging thus leads to thinning of the skin, loss of elasticity and reduction of water content with the formation of skin laxity, wrinkles, and skin corrugations.

Dyschromia and hyperpigmentations of the skin can be added to these signs of skin aging, as a direct consequence of exposure to external agents.

Dermo-cosmetic science aims to limit, prevent or slow down the onset of these manifestations of skin aging, mainly by resorting to the use of biologically active substances aimed at promoting the restoration of the physiological characteristics of the skin such as tone, firmness, hydration, elasticity, brightness, absence of coarseness and roughness.

As part of this task, one of the main objects that cosmetic aims to achieve is to prevent or treat wrinkles, especially facial wrinkles. These consist of a breakdown of skin structures and repeated stretching or extension of certain areas of the skin, especially of the face.

Wrinkle formation has multiple origins being determined by skin aging, gravity, degradation of collagen fibers, loss of elasticity, and exposure to sunlight.

The internal factors are related to the physiological process of aging of the tissues that progressively leads to keratinization of the epidermis, relaxation of the dermis, hypotonia of the "mimic" muscle groups and decreased hydration. Over time, cell turnover in the epidermis becomes increasingly difficult since the outermost area (stratum corneum) becomes drier and thicker, thus impeding cell viability; at the same time the dermis and the deep tissues of the face (including the facial fasciae and muscles) relax causing the loss of tone of the tissues and progressive worsening of wrinkles. Moreover, the progressive loss of efficiency of the skin microcirculation does not allow perfect circulation in the outermost layers of the skin. Many types of cosmetic preparations for external use, which contain combinations of substances with a moisturizing, elasticizing and/or toning action, are currently commercially available.

As an alternative to the external application of cosmetic preparations, fillers are used, which are usually produced in the form of polymer-based gel generally applied in the areas of interest by subcutaneous injection. The most common fillers are based on collagen or hyaluronic acid, in a free or cross-linked form. Their use is intended to replace or supplement the loss of polymers naturally present in the endogenous matrix or to rebalance the function of those polymers in the skin tissue matrix that are still intact.

However, these techniques are expensive and require the intervention of specialized personnel.

At present, therefore, the need is still felt to have new substances for external use that are able to improve the aesthetic appearance of the skin.

Maltodextrins are carbohydrates which for their sweetening properties are mainly used as additives in the food industry and in diet products. Maltodextrins represent suitable replacement for dextrose and are used in the food field, in particular to increase the intake of carbohydrates in the diet without resorting to sugar. Maltodextrins are also used in the pharmaceutical field, within formulations with cicatrizing activity, in particular to treat open wounds such as ulcers, sores resulting from burns as described in US Patent Application 20040001878, in the name of DeRoyal. Maltodextrins are then used as excipients in gel preparations, due to their gel-forming property.

One of the objects of the present invention therefore consists in providing biologically active substances for cosmetic use which are capable of preventing, reducing or delaying the formation of the typical signs of skin aging, such as skin roughness or corrugations.

Another object of the invention is to provide a cosmetic composition that contains an active substance capable of improving the aesthetic appearance of the skin, in particular of the face.

### SUMMARY

The present invention therefore originates from having identified a new use for complex carbohydrates that are typically used as excipients or as active substances in the pharmaceutical field.

Specifically, the invention originates from having found that maltodextrins perform a cosmetic action and improve an aesthetic appearance of the skin when applied to the skin of an individual.

Consequently, according to a first aspect, the present invention concerns the cosmetic use of selected maltodextrins to prevent, reduce or treat the signs of skin aging, wherein advantageously such maltodextrins comprise a fraction of maltodextrins or fragments of maltodextrins having a molecular weight in the range from 600 to 1200 daltons, and a fraction having a molecular weight in the range from 2400 to 3600 daltons.

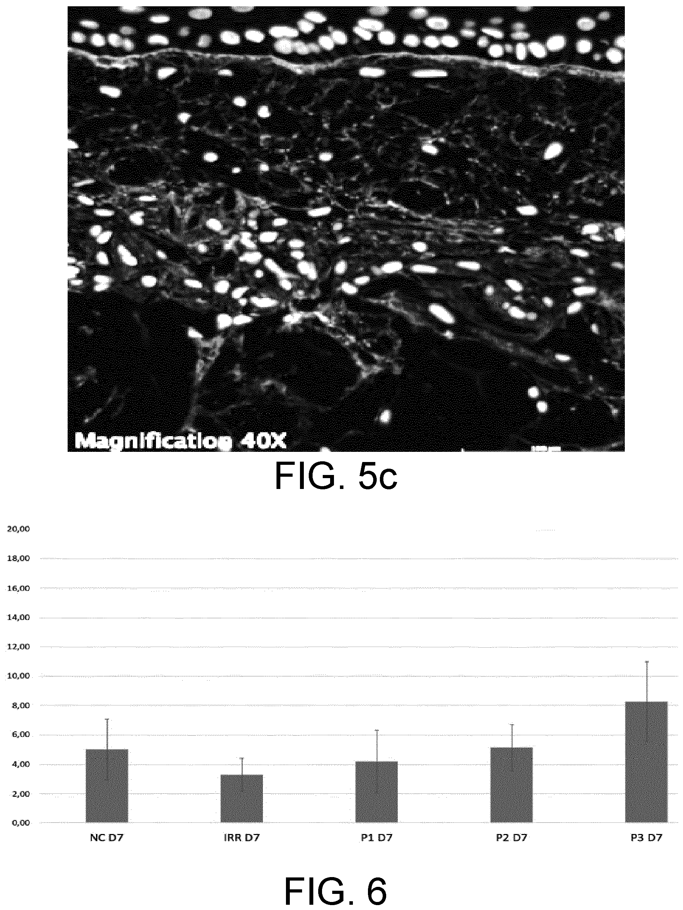

The selected maltodextrins fractions show a greater cosmetic activity than non-selected/purified maltodextrins mixtures, as shown in Example 6, and illustrated in the attached Figure 6.

Within the scope of the present invention, the term purified maltodextrins means maltodextrins with a selected molecular weight as described herein, specifically fractions having a molecular weight in the range from 600 to 1200 daltons and fractions having a molecular weight in the range from 2400 to 3600 daltons.

Within the cosmetic uses of the invention, therefore, the use of these maltodextrins in preventing, reducing or treating facial skin wrinkles is specifically provided for. The inventor has found that these maltodextrins are highly compatible with the epidermis and lead to an improvement in the aesthetic appearance of the skin when applied locally thereon, in a cosmetically effective amount.

According to another aspect, the present invention provides the cosmetic, non-therapeutic use of a cosmetic composition comprising a cosmetically active amount of maltodextrins and a cosmetically acceptable excipient to prevent, reduce or treat the signs of skin aging, in particular of the facial skin.

Preferably the signs of aging treatable with the cosmetic composition of the invention are wrinkles or skin corrugations. The cosmetic composition of the invention therefore finds particular use in the cosmetic treatment of an individual's wrinkles.

### BRIEF DESCRIPTION OF THE FIGURES

Characteristics and advantages of the present invention will be more evident from the appended figures, wherein:

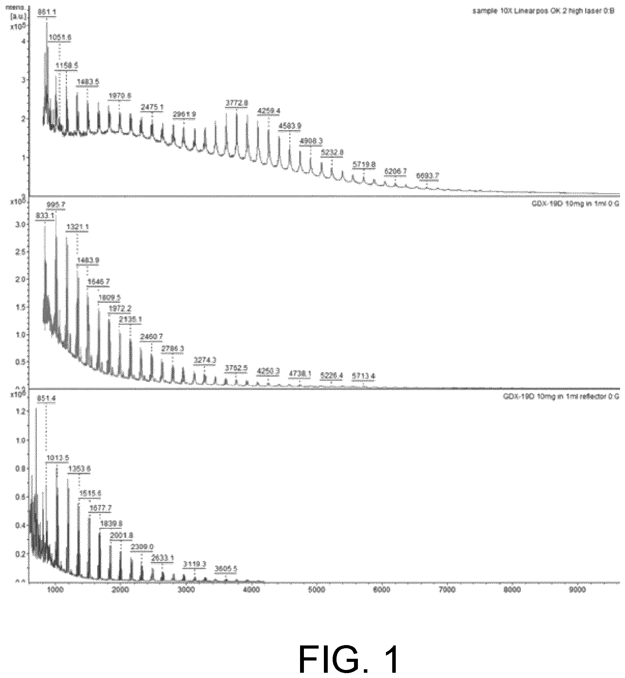

Figure 1 shows the results of a mass spectrometry analysis, wherein the molecular weights of the maltodextrins fractions are expressed in peaks recorded at different times of the manufacturing process of the maltodextrins fractions. Maltodextrins suitable for the purposes of the invention are shown in the bottom image, with the highest number of peaks being related to maltodextrins fragments with molecular weights in the fraction from 600 to 1200 daltons. Starting from the top, the first and second images show the peaks of higher molecular weights of maltodextrins that do not fall within the invention, obtained from purification stages prior to the one resulting in the lower molecular weight fractions, shown in the bottom image;
Figure 2 shows a photographic representation of the fragments with molecular weights of the maltodextrin's fractions. The values shown in the top image are related to maltodextrins obtained at the end of the manufacturing process, suitable for the uses of the invention;
Figure 3 is a higher resolution image of the maltodextrins fraction with presence of higher molecular weights, around about 3000;
Figures 4 a, b, c show 3D microscopy images obtained by immuno-staining of the expression of collagen III protein, both at the level of the reticular dermis and papillary dermis, following treatment of the skin with the composition of Example 6;
Figures 5 a, b, c show 3D microscope images obtained by immuno-staining relative to a negative control (with respect to Figures 4 a, b, c), where it is evident that the gray filamentous portions due to collagen production are lower than those visible in the image of the previous Figures 4 a, b, c;
Figure 6 illustrates the degree of activity of the maltodextrins fraction at various purification levels: P1-P2-P3, where P3 is the mixture object of an embodiment of the invention comprising a fraction of maltodextrins or fragments of maltodextrins having a molecular weight in the range from 600 to 1200 daltons, and a fraction having a molecular weight in the range from 2400 to 3600 daltons.

### DETAILED DESCRIPTION OF THE INVENTION

The present invention originates from having identified that selected fractions of maltodextrin, when applied to the skin, perform a dual moisturizing and filming activity on the skin resulting in a reduction of the typical signs of skin aging. The activity found is cosmetic/aesthetic.

Maltodextrins described herein are complex carbohydrates, typically water soluble. From a structural point of view, maltodextrins comprise D-glucose (dextrose) units connected in chains of variable length. The glucose units are primarily linked with 1-4 α glycosidic bonds.

They are mainly obtained from degradation of natural macromolecules, in particular from cereals starches, for example corn, oats, wheat, rice or tuber starches (potatoes, tapioca), by acid hydrolysis or by enzymatic route.

Typically, maltodextrins are classified based on their "dextrose equivalence" (DE) and this value can range from 3 to 20. The greater is the DE, the shorter are the glucose chains, the greater is the sweetness, the greater is the solubility in water. According to some embodiments, maltodextrins have a molecular weight not higher than 6.000 daltons, preferably a molecular weight from 1.000 to 3.500 daltons. These molecular weights are selected to retain the solubility in water, and therefore the ability to exert also a gelling and filming effect.

Typically, the molecular weights of maltodextrins are expressed as weight average molecular weight MW (Da) and can be determined by using industry standard methods, such as those described in the publications: Ueno et al., 1988, Chem Pharm Bull. 36, 4971-4975; Wyatt 1993, Anal Chim Acta 272: 1-40; Watt Technologies 1999 "Light scattering University Dawn Course Manual and "Dawn Eos Manual" Wyatt Technology Corp. Santa Barbara CA (USA). According to a first aspect of the invention, the use of maltodextrins is therefore provided according to the attached claim 1.

According to certain embodiments, the fraction having a molecular weight in the range from 600 to 1200 daltons includes maltodextrins or maltodextrins fragments comprising 3-6 glucose units, while the fraction having a molecular weight in the range from 2400 to 3600 daltons includes maltodextrins consisting of from 12 to 18 glucose units.

Preferably, the fraction of maltodextrins or related fragments having a molecular weight in the range from 600 to 1200 daltons is equal to 60 - 90% by weight with respect to the total weight, and the fraction having a molecular weight in the range from 2400 to 3600 daltons is equal to 40 - 10% by weight with respect to the total weight.

It was observed that maltodextrins fractions described herein penetrate the hydro-lipid barrier of the skin more easily and perform a moisturizing and nourishing function of the deep and subcutaneous skin tissue.

The applicant has observed that these maltodextrins - when applied to the skin-draw water to the application area and are cosmetically active as they hydrate the epithelial tissue. Furthermore, when applied to the skin as such or in the form of a cosmetic composition, they form a semipermeable membrane that protects the skin from external agents thus contributing to reduce skin dehydration, while keeping perspiration substantially unchanged.

In addition, maltodextrins perform a nourishing action thanks to the sugary nature of the constituent chain, in the absence of adverse effects and in the presence of high biodegradability.

Maltodextrins usable within the scope of the invention can be obtained from starches contained in cereal seeds, as such or possibly sprouted, and in tubers.

For example, they can be obtained starting from starches by:
a. acid hydrolysis, typically using mineral acids such as hydrochloric acid or sulfuric acid at a concentration suitable for determining hydrolysis while limiting denaturation of the fractions obtained. Hydrolysis takes place using conventional techniques, well known to those skilled in the art
b. enzymatic hydrolysis, making use of suitable enzymes such as amylase, glucoamylase. Hydrolysis takes place using conventional techniques, well known to those skilled in the art

After the hydrolysis step, it is preferable to carry out a purification step, for example by ultrafiltration or molecular sieves, and a freeze-drying or drying step, adopting conventional technologies in the field, in order to obtain a solid product that is easy to store and to handle.

The product obtained from the manufacturing methods is preferably a purified water-soluble powder having a homogeneous molecular weight to improve the cosmetic action.

According to some embodiments, selected maltodextrins mixtures comprising from 60 to 90% by weight of a maltodextrins fraction having a molecular weight in the range from 600 to 1200 daltons, and from 40 to 10% by weight of a maltodextrins fraction having a molecular weight in the range from 2400 to 3600 daltons are provided.

Typically, the selected or purified maltodextrins described herein have the following properties:
- a moisturizing and gelling (film-forming) action;
- a stimulating action of fibroblasts and keratinocytes proliferation, responsible for the formation of granulation tissue and producers of collagen, elastin, proteoglycans, and other glycoproteins that intervene in the tissue regeneration phase

- a skin regenerating action, found with representative *in vitro* tests such as synthesis of fibronectin, polymerization of actin, expression of enzymes such as Hyaluronan Synthase-2
- a skin protective action following the formation of an epidermal barrier

For example, in the presence of such maltodextrins it has been found that:
- stimulation of fibroblasts increases by 20% compared to control,
- synthesis of fibronectin shows a 20% increase compared to the control,
- polymerization of actin shows a 200% increase compared to control, whereas the positive standard PA (phosphatidic acid) shows a 100% increase,
- Hyaluronan Synthase-2 expression shows an increase of almost 50%.

The combination of these properties creates a skin microenvironment ideal for the growth of granulation tissue and regeneration with tissue proliferation, which is the basis of the cosmetic effect of the invention.

According to another aspect, the present invention provides a cosmetic composition comprising maltodextrins and a cosmetically acceptable excipient or carrier to prevent or reduce the signs of skin aging.

Herein, the term "carrier" refers to an excipient, carrier, diluent, or coadjuvant that may or not be present in the composition of the invention. A carrier and/or excipient suitable for topical administration of the composition may be used. A particularly suitable carrier is water.

According to some preferred embodiments, the composition does not contain oil, oils, or oil esters.

According to certain embodiments, the composition does not contain a weak carboxylic acid, in particular with a pKa higher than 2, higher than 3, higher than 5. For example, according to these embodiments, the composition does not contain alpha hydroxy acids with a pKa from 2 to 4, monocarboxylic acids with a pKa from 3 to 5, alpha-amino acids with a pKa from 2 to 3. These carboxylic acids confer a pH that reduces or can alter the cosmetic activity of the composition.

Typically, the composition of the invention is used for topical use by application on the face and/or body skin in need of treatment.

The cosmetic composition can be in a solid, semi-solid, or liquid form.

Suitable formulations in a solid form include creams, gels, liniments, pastes, ointments.

Suitable formulations in liquid form include solutions, suspensions, lotions, gels, serums, both oil-in-water and water-in-oil emulsions. In a preferred embodiment, the formulation for local application is in the form of a gel.

In the case of formulations in fluid or semi-fluid form, maltodextrins can be diluted in a liquid form carrier, typically water.

In some embodiments the compositions of the invention can comprise excipients commonly used in the formulation of cosmetic preparations such as preservatives, bactericidal agents, stabilizers, antioxidants, emulsifiers, buffers, humectants, coloring agents and other excipients commonly used in cosmetic preparation techniques, in amounts in line with those provided for common cosmetic formulations.

In case of formulations in the form of solution, suspension, lotion, water is present as a diluent or solvent, optionally mixed with other liquids used for the formulation of cosmetic compositions such as alcohols, and glycols such as propylene glycol.

The composition of the invention may contain the maltodextrins mixture described above in an amount ranging from 0.1% to 80% by weight, based on the chemical-physical characteristics of the product, the type of desired activity, the type and the extension of the area to be treated, the sex and age of the user, as well as the purity of the maltodextrins mixture used.

According to certain embodiments, the composition of the invention further comprises one or more active substances such as minerals, micronutrients, vitamins, and optionally other cosmetic substances.

By way of example, the composition may include one or more additional cosmetically active components selected from amino acids such as arginine, proline, glycine, lysine, polypeptides, hydroxy acids such as lactic, glycolic, salicylic, tartaric, malic acid, etc., natural extracts or plants essential oils, vitamins such as tocopherol, lipoic acid, retinol, panthenol, ascorbic acid, flavonoids etc., glycans such as hyaluronic acid, chondroitin sulfate, heparinoids etc., proteins such as wheat proteins, collagen, silicones, molecules of various nature such as allantoin, caffeine, bisabolol, coenzyme Q10.

A suitable cosmetic plant extract that can be present in the composition of the invention is an extract of *Acmella Oleracea,* containing spilanthol as cosmetically active substance.

According to an aspect of the invention, a cosmetic treatment method is provided to improve the aesthetic appearance of the skin in any area of an individual's body. The cosmetic treatment method comprises the application on the skin of a cosmetically effective amount of a composition containing these maltodextrins and a cosmetically acceptable carrier according to any of the embodiments previously described to improve the aesthetic quality of an anatomical characteristic of the individual.

The improvement of the aesthetic appearance of an anatomical characteristic of an individual includes the improvement of any type of aesthetic quality including the external appearance, the tactile sensation of the skin of any area of the body, preferably the face.

The present invention will now be described with reference to the following examples which are provided for mere illustrative purposes and are not to be intended as limiting the present invention.

### EXAMPLE 1

Cosmetic composition in the form of a base cream containing the following components:
Mixture of selected maltodextrins preferably consisting of 60 to 90% by weight of a maltodextrins fraction (or maltodextrins fragments) having a molecular weight in the range from 600 to 1200 daltons, and from 40 to 10% by weight of a maltodextrins fraction having a molecular weight in the range from 2400 to 3600 daltons, Decyl Oleate (lubricating and emollient oil), White Vaseline (emollient and carrier), Cetearyl Alcohol (emulsifier), Sodium Cetearyl Sulfate (emulsifier), Glycerin (moisturizer), phenoxyethanol and parabens (preservatives), perfume, water (carrier)

### EXAMPLE 2

### Moisturizing Hand and Body Cream:

Mixture of selected maltodextrins preferably consisting of 60 to 90% by weight of a maltodextrins fraction (or maltodextrins fragments) having a molecular weight in the range from 600 to 1200 daltons, and from 40 to 10% by weight of a maltodextrins fraction having a molecular weight in the range from 2400 to 3600 daltons, Octyldodecanol, White Vaseline (emollient and carrier), Glyceryl Stearate (emollient, emulsifier, stabilizer and consistency factor), Glycerin (moisturizer), phenoxyethanol and parabens (preservatives), perfume, water (carrier)

### EXAMPLE 3

### Moisturizing Gel

Mixture of selected maltodextrins preferably consisting of 60 to 90% by weight of a maltodextrins fraction (or maltodextrins fragments) having a molecular weight in the range from 600 to 1200 daltons, and from 40 to 10% by weight of a maltodextrins fraction having a molecular weight in the range from 2400 to 3600 daltons, Hydroxyethylcellulose (thickener and gelling agent), Macrogol 400 (moisturizer and carrier), Glycerin (moisturizer), phenoxyethanol and parabens (preservatives), perfume, water (carrier)

### EXAMPLE 4

### Liquid Detergent:

Mixture of selected maltodextrins preferably consisting of 60 to 90% by weight of a maltodextrins fraction (or maltodextrins fragments) having a molecular weight in the range from 600 to 1200 daltons, and from 40 to 10% by weight of a maltodextrins fraction having a molecular weight in the range from 2400 to 3600 daltons, Sodium Laureth Sulfate, Glycol Distearate (mattifying and pearlizing agent), Cocamides (surfactants), Glycerin (moisturizer), Coco Glucoside (surfactant and surface-active agent), phenoxyethanol and parabens (preservatives), perfume, water (carrier)

### EXAMPLE 5

### Bio-revitalizing Solution:

Mixture of selected maltodextrins preferably consisting of 60 to 90% by weight of a maltodextrins fraction (or maltodextrins fragments) having a molecular weight in the range from 600 to 1200 daltons, and from 40 to 10% by weight of a maltodextrins fraction having a molecular weight in the range from 2400 to 3600 daltons, sodium chloride (isotonicizing agent), water (carrier).

### EXAMPLE 6

Structural characteristics of a maltodextrins mixture preferentially (but not exclusively) consisting of 60 to 90% by weight of a maltodextrins fraction (or maltodextrins fragments) having a molecular weight in the range from 600 to 1200 daltons, and from 40 to 10% by weight of a maltodextrins fraction having a molecular weight in the range from 2400 to 3600 daltons.

In order to verify the degree of purity and especially the average molecular weight of the obtained fraction, mass spectrometry analyzes were performed under controlled conditions. In this type of technique, it is possible to identify the molecular weights of the molecular species present in the sample under analysis, as well as the related main fragmentation peaks.

### Characteristics of the maltodextrins fraction:

As shown in Figure 1, peaks are representative of the state of the maltodextrins fraction at different stages of purification. In the maltodextrins obtained at the end of the manufacturing process, suitable for the uses of the invention, the fraction with molecular weights ranging from 600 to 1200 daltons is mostly represented, while other images in Figure 1 show the peaks in the previous purification stages which highlight the presence of maltodextrins with higher molecular weights.

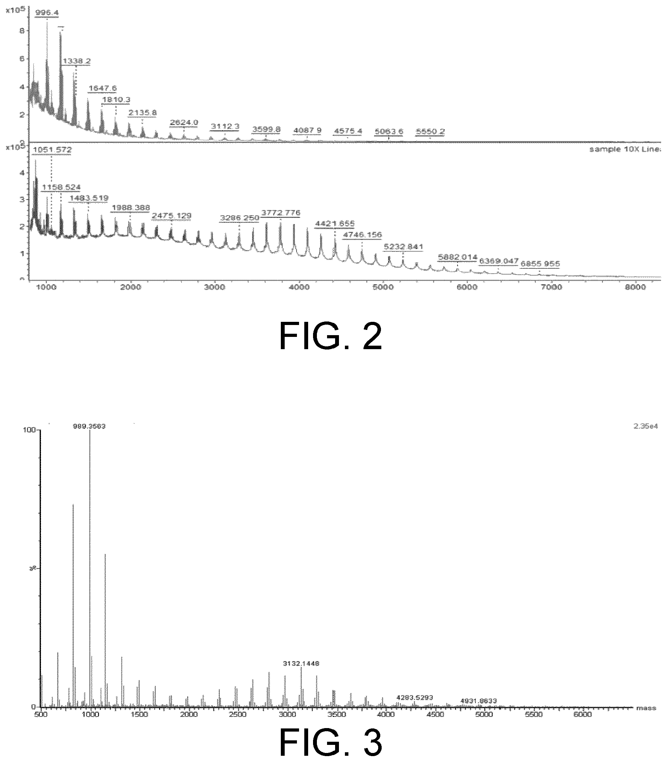

In Figure 2, the values shown in the top image relate to maltodextrins obtained at the end of the manufacturing process, suitable for the uses of the invention; at the bottom, the previous purification stage is visible.

By expanding the sensitivity of the method, it is highlighted that fractions with higher molecular weights, around 3000, are present in the final fraction, as shown in Figure 3.

The experimental data show that the tested mixture comprises a prevalent fraction having a molecular weight in the range from 600 to 1200 daltons and a fraction having a higher molecular weight in the range 2400-3600 daltons. Polysaccharides with a MW higher than 3600 are not significantly present in the mixture.

### EXAMPLE 7

### Photoaging treatment using maltodextrins fractions of Example 6

In order to verify the activity of the selected maltodextrins fractions with respect to the non-purified mixture, an *in vitro* photoaging test was performed, which allows to predict the effectiveness of products against advancing age and especially sun damage, particularly UV rays, through photo-induction and immunofluorescence measurement. About this test following explanatory details are provided.

By using this method, it is possible, *inter alia,* to measure the intensity of cellular reproduction and gene expression of proteins essential for the dermal tissue.

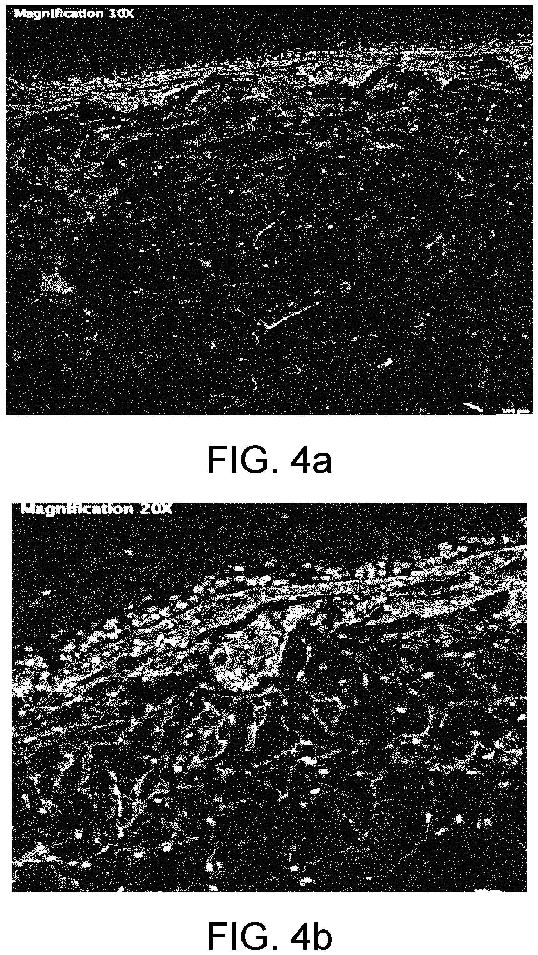

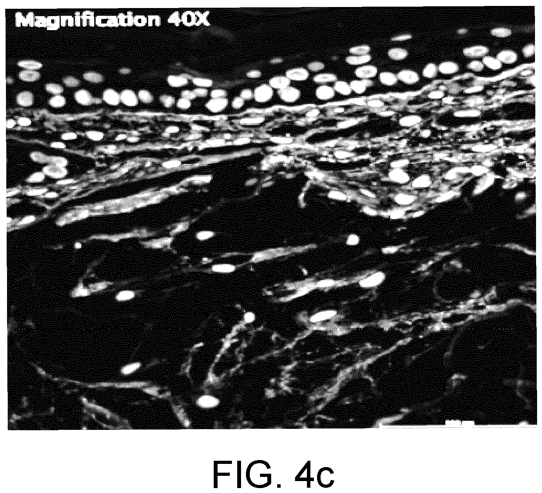

The fraction in aqueous solution and without addition of any other component denoted a very remarkable expression of collagen III protein (gray filamentous material) both at the level of reticular dermis and papillary dermis (positioned immediately below the epidermis) as visible under 3D microscope in the images of Figures 4 a, b, c obtained by immuno-staining, and to a more evident extent in the larger image (magnification 40x): the gray filamentous material is represented by the fluorescent signal due to collagen III developed by the product. The nuclei (light discoidal), not significant for the purpose of the activity, appear in light gray. Collagen III is a vital protein material for the dermis, and also represents the anchorage site for other collagens; it is therefore vital for skin restructuring.

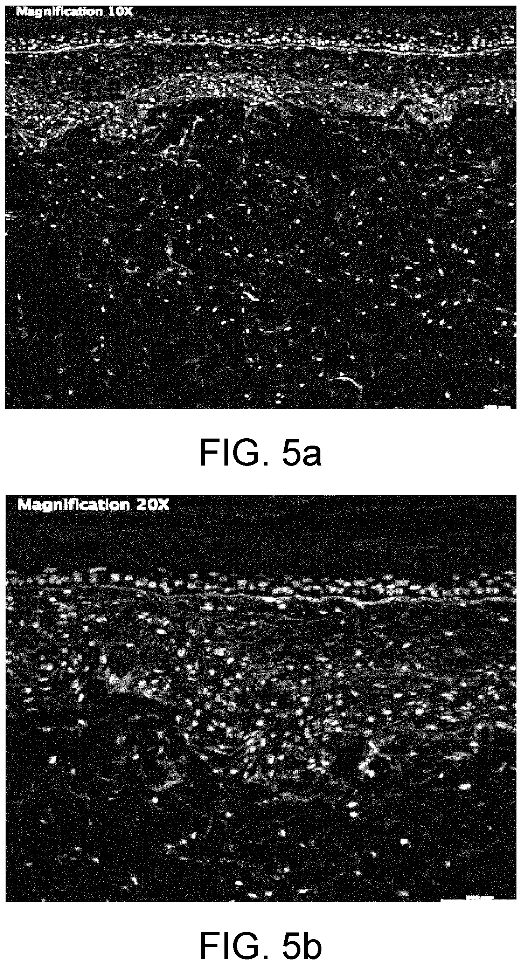

For comparative purposes, the effect of a negative control (placebo) is provided in the images of Figures 5 a, b, c, where it is evident that the gray filamentous material - due to production of collagen - is much less than that visible in the images of the previous Figures 4 a, b, c.

In the image of Figure 6, it is shown the degree of activity of the maltodextrins fraction at various stages of purification, P1-P2-P3, where P3 is the final mixture of Example 6.

### EXAMPLE 8

### Experimental Test

In order to verify some cosmetic properties, tests were performed on 30 healthy volunteers, under controlled conditions and according to standard procedures for cosmetic testing, using the composition in the form of a cream containing the maltodextrins of Example 1.

The tests were performed in comparison to a placebo and a positive standard consisting of a market-leading moisturizer.

The results highlighted the following effects:
a. Moisturizing effect: at 30 minutes and 3-6-24 hours after application, the cream containing the selected maltodextrins has a moisturizing effect far superior to the placebo in the immediate checks and then in a percentage not lower than +20% in the other checks performed over the day. Compared to the standard, the cream containing the maltodextrins showed a moisturizing effect in average 10% higher
b. Regenerating and soothing effect after skin erythema induced by radiation: a 1-2-3-4 days after the first application, the cream containing the selected maltodextrins - applied once a day - causes a regression of erythema higher than placebo, with an increasing value as a function of the check time in the immediate checks, and up to a value of less than -20% at the end of the day 4, and in values comparable to the positive standard
c. Regenerating and protective effect after slight removal of the stratum corneum: at 10'-30'-3h-6h after application, the cream containing the selected maltodextrins causes a regenerating and protective effect (measured by checking the TEWL - Trans Epidermal Water Loss - used as an index of skin tissue regeneration that prevents water loss), in values that reach more than + 50% compared to the placebo, and in values comparable with the positive standard.

### EXAMPLE 9

### Intradermal application of the composition

The solution of the maltodextrins of Example 5 was applied by intradermal infiltrations performed locally in the most sensitive areas of the body such as face, neck, back of the hands in 10 subjects with signs of skin aging.

This treatment resulted in a "positive" to "very positive" evaluation both by the patient and the doctor, in terms of tone, firmness, coloring of the skin, and improvement of expression wrinkles; as objective parameters, the degree of skin hydration as well as skin elasticity have increased in at least 75% of cases. The intradermal treatment was free of adverse effects, thanks to biodegradability of the maltodextrins chemical structure. This treatment was safer than intradermal treatments with hyaluronic acid, particularly of the cross-linked and/or high molecular weight type.

### EXAMPLE 10

### Maltodextrins and plant extract combination

A cream based on a combination of selected maltodextrins and an extract of *Acmella Oleracea* was tested to verify cosmetic and anti-wrinkle properties thereof; the tests were performed in comparison to a placebo.

The results show how the cream makes the skin smoother, radiant and hydrated, with values higher than 80% based on the judgment of the user and the investigator, where the placebo does not exceed 30-40%; at the same time, the cream also showed a significant reduction in wrinkles and an increase in skin elasticity of over 90% after 8 weeks of treatment, whereas the placebo values do not exceed 30-40%.

### EXAMPLE 11

### Preparation of a maltodextrins embodiment for the uses of the invention.

The desired maltodextrins fraction is obtained from a crude maltodextrins mixture made according to traditional methods.

The crude mixture, for example of the type used for food purposes, includes maltodextrins having molecular weights varying over a wide range of molecular weight (MW) starting from minimum sizes up to tens of thousands of daltons. Depending on the manufacturing process, these mixtures include very low MW carbohydrate impurities (down to simple mono and disaccharides, such as glucose, maltose, sucrose etc.) and, on the other hand, high MW impurities, even higher than 10,000-30,000 daltons, which are not very water-soluble and contribute to give a cloudy appearance to the solution in water.

A suitable maltodextrins fraction is obtained by "cutting" the high MW molecules, for example by means of ultrafiltration membranes.

The initial mixture is first dissolved; then ultrafiltration is performed using membranes with a 5,000 (or 10,000) cut-off, washing repeatedly until the eluate is "white", i.e. until there is significant passage of substances with a MW lower than the cut-off. The phase retained by the filter, consisting of the retained MW, is therefore discharged.

Subsequently, a similar operation is performed by passing the eluate previously obtained through another membrane with a 3,000 cut-off: in this case, the lower MW molecules are eluted and discharged, until the eluate becomes "white".

The cut-off values indicated, 3,000 and 5,000 (10,000) refer - for ultrafiltration membranes on the market - to globular, and therefore non-linear and without ramifications, protein molecules.

In the event that these membranes are used with linear and branched molecules, such as maltodextrins consisting of long saccharide chains, the effective "cut" is significantly lower due to the spatial dimension of these molecules.

## Claims

1. Cosmetic use of maltodextrins comprising a maltodextrins fraction having a molecular weight in the range from 600 to 1200 daltons and a maltodestrin fraction having a molecular weight in the range from 2400 to 3600 daltons to prevent, reduce or treat the signs of skin aging.

2. The cosmetic use according to claim 1, wherein the maltodextrins fraction having a molecular weight from 600 to 1200 daltons include 3-6 glucose units and the fraction having a molecular weight from 2400 to 3600 daltons includes from 12 to 18 glucose units.

3. The cosmetic use according to claim 1, wherein the fraction having a molecular weight from 600 to 1200 daltons is from 60 to 90% by weight and the fraction having a molecular weight from 2400 to 3600 daltons is from 40 to 10% by weight.

4. Use of a cosmetic composition comprising a cosmetically active amount of a maltodextrins fraction having a molecular weight in the range from 600 to 1200 daltons, and optionally a maltodextrins fraction having a molecular weight in the range from 2400 to 3600 daltons, and an aqueous-based carrier to prevent, reduce or treat the signs of skin aging and/or prevent or reduce the depth of wrinkles or skin corrugations.

5. The use according to claim 4, wherein the carrier is water.

6. The use according to claim 4 or 5, wherein the maltodextrins fraction having a molecular weight from 600 to 1200 daltons includes 3-6 glucose units and the fraction having a molecular weight from 2400 to 3600 daltons includes from 12 to 18 glucose units.

7. The use according to claims 4 - 6, wherein the composition does not contain oil.

8. The use according to any one of claims 4 - 7, wherein said composition comprises at least one further component selected from amino acids, polypeptides, natural extracts or plants essential oils, vitamins flavonoids, glycans, proteins, collagen, silicones, allantoin, caffeine, bisabolol, coenzyme Q10, and mixtures thereof.

9. The use according to claim 8, wherein the amino acids are selected from arginine, proline, glycine, lysine, and mixtures thereof.

10. The use according to any one of claims 4 - 9 further comprising an extract of *Acmella Oleracea.*
